(19) Europäisches Patentamt
European Patent Office
Office européen des brevets

(11) **EP 4 144 334 A1**

(12) **EUROPEAN PATENT APPLICATION**

(43) Date of publication:
08.03.2023 Bulletin 2023/10

(21) Application number: 22192422.8

(22) Date of filing: 26.08.2022

(51) International Patent Classification (IPC):
*A61K 6/15* (2020.01)   *A61K 6/78* (2020.01)
*A61K 6/836* (2020.01)   *A61K 6/887* (2020.01)
*A61K 6/64* (2020.01)   *A61K 6/76* (2020.01)
*A61K 6/77* (2020.01)   *A61K 6/17* (2020.01)

(52) Cooperative Patent Classification (CPC):
(C-Sets available)
A61K 6/15; A61K 6/17; A61K 6/64; A61K 6/76;
A61K 6/77; A61K 6/78; A61K 6/887        (Cont.)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 01.09.2021 CN 202111019441

(71) Applicant: Aidite (Qinhuangdao) Technology Co.,
Ltd.
Qinhuangdao Hebei 066004 (CN)

(72) Inventors:
• ZHANG, Jiaxin
Qinhuangdao, 066004 (CN)
• LIU, Qianqian
Qinhuangdao, 066004 (CN)
• QIAO, Chunmei
Qinhuangdao, 066004 (CN)

(74) Representative: Ulrich, Thomas Franz et al
Gleiss Große Schrell & Partner mbB
Patentanwälte Rechtsanwälte
Leitzstraße 45
70469 Stuttgart (DE)

(54) **DENTAL GRADIENT COLOR-RESIN CERAMIC RESTORATION MATERIAL AND PREPARATION METHOD THEREOF**

(57)    Disclosed are a dental gradient color-resin ceramic restoration material and a preparation method thereof. The method includes: preparing at least two paste materials with different colors, respectively dispersing the paste materials with different colors to obtain at least two granular materials with different colors; spreading the at least two granular materials with different colors to be flat in a mould in sequence to obtain at least two layers, and then tightly combining the at least two layers to obtain a semi-finished product of a gradient color-resin ceramic restoration material; polymerizing and curing the semi-finished product to obtain a multilayer gradient color-resin ceramic restoration material.

EP 4 144 334 A1

(52) Cooperative Patent Classification (CPC): (Cont.)

C-Sets
**A61K 6/887, C08L 33/10**

**Description**

**TECHNICAL FIELD**

The present invention belongs to the technical field of medical materials, and relates to a dental gradient color-resin ceramic restoration material and a preparation method thereof.

**BACKGROUND ART**

With the rapid development of Computer Aided Design/Computer Aided Manufacturing (CAD/CAM) technology, people raise higher and higher requirements for the speed and performance of dental restoration. At present, there are mainly two kinds of machinable dental restoration materials matched with CAD/CAM technology system, i.e., ceramic materials and resin restoration materials. The two kinds of materials have their own advantages and disadvantages in dental restoration. Ceramic materials have high mechanical and aesthetic properties, but have great damage to natural teeth due to their brittleness and high wear resistance. Resin materials are still widely used in CAD/CAM technology due to their low price, good toughness, easy machining, short restoration period, and no abrasion to paired jaw teeth. However, resin materials could not be used for permanent restoration because of their disadvantages of low strength, poor wear resistance and short service life. Resin ceramic restoration materials are novel dental restoration materials, and their performance is closer to that of natural teeth. Resin ceramic restoration materials not only have the characteristics of ceramic materials such as excellent biocompatibility, hardness and thermal stability, but also have the characteristics of resin materials such as elasticity, thus effectively solving the problem of brittle fracture of ceramic materials, improving the brittleness and wear resistance of ceramic materials, and improving the mechanical properties of resin materials.

However, natural teeth have a gradient color. From incisal portion to cervial portion of the teeth, the color gradually deepens, and the transparency gradually decreases. Aesthetic effect of monochromatic resin ceramic restoration materials could not meet the needs of patients, thus requiring multi-layer gradient color-resin ceramic restoration materials.

WO 2009/154301 A1 discloses a method for manufacturing a dental prosthesis, which comprises partially filling a resin material which contains an inorganic filler into a mold with an inner surface shaped in an outer shape of a block, and then performing rotational agitation on the resin material, and after stirring to be uniform, polymerizing and curing the resin material, and repeating the above process to obtain a composite resin block with color gradients. However, since each layer is polymerized and cured independently, a laminated structure is formed by re-polymerization, which easily results in obvious boundary between layers and weak interlayer combining.

WO 2018/074583 A1 discloses a method for preparing a multilayer composite resin material, which comprises uniformly mixing raw materials such as a polymerizable monomer, an inorganic filler initiator, and a colorant, to obtain pastes with different colors, respectively introducing three pastes with different colors into three cylinder containers, and simultaneously introducing the three pastes with different colors into a mold through filling nozzles. During the filling, the material of the outer layer flows by pressing the central layer, and the material of the central layer is filled by diffusing towards the outer layer. In the composite material block filled into the mold by this means and shaped, there is no bubbles in the boundary layer, and the three layers have almost uniform thickness. However, this method requires the prepared pastes to have high fluidity, which limits the content of inorganic filler and the ratio of different polymerizable monomers, resulting in poor mechanical properties. In addition, natural teeth have a nonuniform thickness distribution between layers with different colors, while the multilayer composite resin material prepared by this method has an almost uniform thickness distribution between the three layers, which is inconsistent with the aesthetic effect of natural teeth.

CN 105362084 A discloses a method for preparing multilayer-colored dental composite materials, which comprises mixing a polymer monomer matrix, an inorganic filler, an initiator, a coloring agent, and a grinding additive, and milling for 0.5-2 h, and then drying to obtain monochromatic composite precursor powders; adding composite precursor powders with different colors into a dry-pressing mould in sequence, and then pre-moulding at a pressure of 20-30 MPa to obtain a pre-moulded green body; pressurizing and heating the pre-moulded green body of multilayer-colored composite materials to obtain the multilayer-colored dental composite materials. In this preparation method, a volatile organic solvent is added as a grinding additive such that the polymer monomer matrix, the inorganic filler, the initiator and the coloring agent could be evenly mixed; after the material is ground to be uniform, it is necessary to remove the volatile grinding additive by drying. This preparation method involves a complicated process, and has disadvantage of easy remain of the grinding additive, which leads to the decrease of the performance of the composite materials.

In conclusion, at present, many multilayer gradient color-resin ceramic restoration materials have problems such as obvious boundary between layers, poor mechanical strength and complicated preparation process. It is urgent to develop a resin ceramic restoration material which has natural gradual change effect without layering, enough mechanical strength to bear occlusal pressure in oral cavity and simple preparation process.

## SUMMARY

In view of the above, the present invention is to provide a dental gradient color-resin ceramic restoration material and a preparation method thereof. The resin ceramic restoration material of the present invention has natural gradual change effect without layering, and enough mechanical strength to bear occlusal pressure in oral cavity.

To achieve the above objects, the present invention provides the following technical solutions:

The present invention provides a method for preparing a dental gradient color-resin ceramic restoration material, comprising:

step 1, preparing at least two paste materials with different colors, and respectively dispersing the paste materials with different colors to obtain at least two granular materials with different colors;

step 2, spreading the at least two granular materials with different colors to be flat in a mould in sequence to obtain at least two layers, and then tightly combining the at least two layers to obtain a semi-finished product of a gradient color-resin ceramic restoration material; and

step 3, polymerizing and curing the semi-finished product obtained in the step 2 to obtain a multilayer gradient color-resin ceramic restoration material.

According to the present invention, in the above preparation method, granular materials are firstly obtained, and then pressed and cured to form color gradient layers between different color layers, resulting in a natural gradient change effect without obvious layering, and good mechanical strength.

In some embodiments, each of the paste materials is independently prepared by a process comprising:

adding a polymerizable monomer, an initiator, an inorganic filler and a colorant into a mixing device, and stirring to be uniform to obtain the paste material; or

adding a polymerizable monomer, an initiator, an inorganic filler and a colorant into a mixing device in batches, and stirring to be uniform to obtain the paste material.

In some embodiments, the at least two granular materials with different colors obtained in step 1 each independently have a particle size of 0.1 mm to 5 mm, such as 0.1 mm, 0.3 mm, 0.5mm, 1 mm, 2 mm, 3 mm, 4 mm or 5 mm.

In some embodiments, the dispersing in step 1 is conducted at a rotating speed of 20 r/min to 150 r/min, such as 20 r/min, 25 r/min, 30 r/min, 40 r/min, 50 r/min, 70 r/min, 90 r/min, 100 r/min, 120 r/min, and 150 r/min, and preferably 60 r/min to 120 r/min.

In some embodiments, the dispersing in step 1 is conducted under a vacuum degree of -0.01 MPa to -0.1 MPa, such as -0.01 MPa, -0.03 MPa, -0.05 MPa, -0.08 MPa or -0.1 MPa, and preferably -0.07 MPa to -0.09 MPa.

In some embodiments, the tightly combining the at least two layers in step 2 is conducted at a pressure of 0.5 MPa to 5 MPa, such as 0.5 MPa, 0.8 MPa, 1 MPa, 1.5 MPa, 2 MPa, 2.5 MPa, 3 MPa, 3.5 MPa, 4 MPa, 4.5 MPa or 5 MPa, and preferably 1 MPa to 3 MPa.

In some embodiments, the polymerizing and curing in step 3 is conducted at a pressure greater than that of the tightly combining the at least two layers in step 2, and preferably 2 MPa to 50 MPa, such as 3 MPa, 5 MPa, 8 MPa, 10 MPa, 15 MPa, 18 MPa, 20 MPa, 25 MPa, 28 MPa, 30 MPa, 35 MPa, 40 MPa, 45 MPa or 50 MPa, and further preferably 10 MPa to 30 MPa.

In some embodiments, the polymerizing and curing in step 3 is conducted at a temperature of 60 °C to 200 °C, such as 60 °C, 70 °C, 80 °C, 90 °C, 100 °C, 120 °C, 150 °C, 180 °C. or 200 °C, and preferably 100 °C to 150 °C.

In some embodiments, a weight ratio of the polymerizable monomer to the inorganic filler in raw materials for preparing the at least two paste materials with different colors is in a range of 10: 90 to 90: 10, such as 10: 90, 15: 85, 20: 80, 22: 78, 25: 75, 30: 70, 40: 60, 50: 50, 60: 40, 70: 30, 80: 20, and 90: 10, and preferably 15: 85 to 40: 60.

In some embodiments, a weight percentage of the initiator to a total amount of the polymerizable monomer and the inorganic filler is in a range of 0.01 wt% to 10 wt%, such as 0.01 wt%, 0.05 wt%, 0.08 wt%, 0.1 wt%, 0.8 wt%, 1 wt%, 2 wt%, 3 wt%, 4 wt%, 5 wt%, 6 wt%, 7 wt%, 8 wt%, 9 wt%, or 10 wt%, and preferably 0.1 wt% to 5 wt%.

In some embodiments, a weight percentage of the colorant to a total amount of the polymerizable monomer and the inorganic filler is in a range of 0.01 wt% to 3 wt%, such as 0.01 wt%, 0.05 wt%, 0.08 wt%, 0.1 wt%, 0.5 wt%, 0.8 wt%, 1 wt%, 1.5 wt%, 2 wt%, 2.5wt% or 3 wt%, and preferably 0.1 wt% to 1 wt%.

In some embodiments, raw materials for preparing the at least two paste materials with different colors further comprise an additive. In some embodiments, the additive is at least one selected from the group consisting of an accelerator, a crosslinking agent, a fluorescent agent, a polymerization inhibitor, an antibacterial agent, an ultraviolet absorber, an anti-tarnishing agent, a viscosity regulator, a wetting agent, an antioxidant, a stabilizer, and a diluent.

In some embodiments, a weight percentage of the additive to a total amount of the polymerizable monomer and the inorganic filler is in a range of 0 wt% to 5 wt%, such as 0.01 wt%, 0.05 wt%, 0.08 wt%, 0.1 wt%, 0.8 wt%, 1 wt%, 2 wt%, 3 wt%, 4 wt% or 5 wt%, and preferably 0.01 wt% to 1 wt%.

In some embodiments, the polymerizable monomer is a well-known free radical resin monomer which could be used in dental materials without any limitation. In some embodiments, the suitable free radical resin monomer comprises at least one selected from the group consisting of a monofunctional group-monomer compound, a bifunctional group-monomer compound, and a monomer compound with at least three functional groups.

In some embodiments, the monofunctional group-monomer compound includes, but is not limited to, at least one selected from the group consisting of methyl methacrylate, ethyl methacrylate, isobutyl methacrylate, tert-butyl methacrylate, iso-amyl methacrylate, benzyl methacrylate, glycidyl methacrylate, dodecyl methacrylate, tetrahydrofurfuryl methacrylate, 2-(N,N-dimethylamino) ethyl methacrylate, 2,3-dibromopropyl methacrylate, 2-hydroxyethyl methacrylate, 2-hydroxypropyl methacrylate, 6-hydroxyhexyl methacrylate, 10-hydroxydecyl methacrylate, triethylene glycol monomethacrylate, propylene glycol monomethacrylate, ethylene glycol methacrylate, diethylene glycol methacrylate, methoxy diethylene glycol methacrylate, polyethylene glycol methacrylate, N-methylol methacrylamide, N-succinyl methyl acrylamide, and 10-methacryloyloxydecyl dihydrogen phosphate.

In some embodiments, the bifunctional group-monomer compound includes, but is not limited to, at least one selected from the group consisting of ethylene glycol dimethacrylate, propylene glycol dimethacrylate, neopentanediol dimethacrylate, butanediol dimethacrylate, hexanediol dimethacrylate, poly(ethylene glycol) dimethacrylate, triethylene glycol dimethacrylate, urethane dimethacrylate, bisphenol A glycerolate dimethacrylate (2,2-bis(4-(3-methacryloyloxy-2-hydroxypropoxy)phenyl)propane), 2,2-bis(4-methacryloyloxyethoxyphenyl)propane, 2,2-bis(4-methacryloyloxydiethoxyphenyl)propane, and 2,2-bis(4-methacryloyloxypolyethoxyphenyl)propane.

In some embodiments, the monomer compound with at least three functional groups includes, but is not limited to, at least one selected from the group consisting of trimethylolpropane trimethacrylate, trimethylolethane trimethacrylate, trimethylolpropane trimethacrylate, tetramethylolmethane trimethacrylate, pentaerythritol tetramethylacrylate, bis-trimethylolpropane tetramethacrylate, dipentaerythritol tetramethacrylate, dipentaerythritol hexamethacrylate, and N,N'-(2,2,4-trimethylhexamethylene)bis(2-(aminocarboxyl)propane-1,3-diol) tetramethylacrylate.

In some embodiments, the polymerizable monomer after polymerizing has a refractive index of 1.52 to 1.58, such as 1.52, 1.53, 1.54, 1.55, 1.56, 1.57 or 1.58, and preferably 1.53 to 1.55.

In some embodiments, the inorganic filler includes an inorganic filler A and an inorganic filler B, wherein the inorganic filler A has an average particle size of 0.1 $\mu$m to 10 $\mu$m, such as 0.5 $\mu$m, 0.8 $\mu$m, 1 $\mu$m, 2 $\mu$m, 3 $\mu$m, 4 $\mu$m, 5 $\mu$m, 6 $\mu$m, 7 $\mu$m, 8 $\mu$m, 9 $\mu$m or 10 $\mu$m, and the inorganic filler B has an average particle size of 10 nm to 40 nm, such as 10 nm, 15 nm, 18 nm, 20 nm, 23 nm, 25 nm, 28 nm, 30 nm, 35 nm, 38 nm, or 40 nm.

In some embodiments, the inorganic filler A includes at least one selected from the group consisting of silica, aluminum silicate, aluminium oxide, titanium dioxide, zirconium oxide, glass (including fluorine glass, borosilicate glass, sodium glass, barium glass, barium aluminum silicate glass, strontium or zirconium containing glass, glass ceramic, fluoroaluminosilicate glass, synthetic glass obtained by sol-gel method, and so on), fumed silica, calcium fluoride, strontium fluoride, calcium carbonate, kaolin, clay, mica, aluminum sulfate, calcium sulfate, barium sulfate, titanium oxide, calcium phosphate, hydroxyapatite, calcium hydroxide, strontium hydroxide, and zeolite. These inorganic fillers may be used as aggregates, such as a silica-zirconium oxide composite oxide aggregate obtained by mixing silica sol and zirconium oxide sol, and subjecting the resulting mixture to a spray drying and a heat treatment.

In some embodiments, the inorganic filler B includes at least one selected from the group consisting of silica, zirconium oxide, titanium oxide, zirconium oxide, zinc oxide, calcium phosphate, and hydroxyapatite.

In some embodiments, the inorganic filler A has a refractive index of 1.52 to 1.58, such as 1.52, 1.53, 1.54, 1.55, 1.56, 1.57 or 1.58, and the inorganic filler B has a refractive index of 1.43 to 1.50, such as 1.43, 1.45, 1.48, or 1.50.

In some embodiments, in order to improve the affinity between the inorganic filler and the polymerizable monomer, and improve the chemical bonding between the inorganic filler and the polymerizable monomer, so as to improve the mechanical strength of cured product, the inorganic filler is subjected to a surface treatment with a surface treatment agent (such as a coupling agent). In some embodiments, the surface treatment agent is at least one selected from the group consisting of $\gamma$-methacryloxy propyl trimethoxyl silane, $\gamma$-methacryloxypropyltriethoxysilane, and $\gamma$-aminopropyltrimethoxysilane, and preferably $\gamma$-methacryloxy propyl trimethoxyl silane.

In some embodiments, the surface treatment with the surface treatment agent is performed by a process comprising: heating a solution containing the inorganic filler, the surface treatment agent and a solvent (such as alcohol) for dozens of minutes to about 10 hours, preferably 1 hour to 5 hours, during which a recycling method may be used. If it is necessary to promote hydrolysis of the surface treatment agent, water or an acidic liquid (such as acetic acid) is added into the solvent, and the resulting mixture is heated and recycled for the above period, and then the solvent is removed by means such as drying under normal pressure or reduced pressure.

An amount of the surface treatment agent varies depending on the particle size of the inorganic filler. In some embodiments, the surface treatment agent is used in an amount of 0.1-50 parts by weight, preferably 1-30 parts by weight, based on 100 parts by weight of the inorganic filler. The filler after the surface treatment has almost the same particle size or particle size distribution as that of the filler before the surface treatment.

In some embodiments, the initiator is at least one selected from the group consisting of dicumyl peroxide, tert-butyl peroxide, benzoyl peroxide, tert-butyl peroxyacetate, and tert-butyl peroxybenzoate.

In some embodiments, the colorant is at least one selected from the group consisting of zinc white, titanium dioxide, antimony oxide, vermilion, cadmium red, iron(III) oxide, chromium oxide, cerium praseodymium yellow, lemon yellow, lead sulfochromate yellow, zinc chrome yellow, iron oxide yellow, bismuth yellow, barium chromate, zirconium vanadium yellow, iron oxide black, carbon black, and graphite.

In some embodiments, the mixing device is a well-known mixing device such as a stirred tank, a kneader, and a mixer.

In some embodiments, the at least two granular materials with different colors obtained in step 1 need to be stored at a temperature lower than 35 °C.

On the other hand, the present invention provides a dental gradient color-resin ceramic restoration material prepared by the above method. The dental gradient color-resin ceramic restoration material has natural gradient change effect without layering, and enough mechanical strength to bear occlusal pressure in oral cavity.

Compared with the prior art, the embodiments of the present invention have the following beneficial effects:

The resin ceramic restoration material prepared by the method of the present invention has natural interlayer transition effect without interlayer layering, enough mechanical strength to bear occlusal pressure in oral cavity, simple preparation process and easy operation.

## DETAILED DESCRIPTION OF THE EMBODIMENTS

The technical solution of the present invention will be further illustrated by the following specific embodiments. It should be understood to those skilled in the art that the described embodiments are only to help understand the present invention, and should not be regarded as specific limitation of the present invention.

The following shows compounds used in the examples of the present invention and abbreviated symbols thereof.

UDMA: urethane dimethacrylate
Bis-GMA: 2,2-bis(4-(3-methacryloyloxy-2-hydroxypropoxy) phenyl) propane
TEGDMA: triethylene glycol dimethacrylate
BPO: benzoyl peroxide
γ-MPS: γ-methacryloxy propyl trimethoxyl silane
Inorganic filler:

1: amorphous barium boroaluminosilicate glass powder GM27884, 0.7 μm (D50);
2: amorphous barium boroaluminosilicate glass powder GM27884, 0.18 μm (D50); and
3: fumed silica OX-50, 40 nm (D50).

## Example 1

(1) Amorphous barium boroaluminosilicate glass powder GM27884 with a D50 of 0.7 μm and fumed silica OX-50 with a D50 of 40 nm were modified by using γ-MPS, respectively, to obtain a modified glass powder and a modified fumed silica.

(2) 70 g of UDMA and 30 g of TEGDMA were added with 1.0 g of BPO as a thermal initiator, to obtain a polymerizable monomer composition.

(3) According to the formulation in Table 1, the modified filler, the polymerizable monomer composition, colorants and an additive were mixed to be uniform to obtain paste materials with different colors.

(4) The paste materials with different colors were respectively stirred for 20 min at a rotating speed of 100 r/min under a vacuum degree of -0.09 MPa, to obtain granular materials with an average particle size of 1.2 mm.

(5) The granular materials were weighted for each layer according to a color transition sequence, then introduced into a press-forming mould in sequence, and spread to be flat. A pressure of 2 MPa was applied to the mould to combine layers tight, to obtain a semi-finished product of a multilayer gradient color-resin ceramic restoration material.

(6) The semi-finished product of the multilayer gradient color-resin ceramic restoration material obtained in step (5) was pressed at 120 °C and 10 MPa for 2 h for polymerizing and curing, obtaining the multilayer gradient color-resin ceramic restoration material.

Table 1

| Number of layers | Modified glass powder | Modified fumed silica | Polymerizable monomer composition | Titanium dioxide | Iron(III) oxide | Lead sulfochromate yellow | Iron oxide black |
|---|---|---|---|---|---|---|---|
| 1 | 67.41457% | 7.49051% | 24.96836% | 0.02327% | 0.03916% | 0.04938% | 0.01474% |
| 2 | 67.39639% | 7.48849% | 24.96163% | 0.02822% | 0.04750% | 0.05989% | 0.01788% |
| 3 | 67.37823% | 7.48647% | 24.95490% | 0.03317% | 0.05583% | 0.07039% | 0.02102% |
| 4 | 67.36006% | 7.48445% | 24.94817% | 0.03812% | 0.06415% | 0.08089% | 0.02415% |
| 5 | 67.34191% | 7.48243% | 24.94145% | 0.04306% | 0.07247% | 0.09138% | 0.02728% |

**Example 2**

(1) Amorphous barium boroaluminosilicate glass powder GM27884 with a D50 of 0.7 $\mu$m was modified by using $\gamma$-MPS,to obtain a modified glass powder.

(2) 70 g of UDMA and 30 g of TEGDMA were added with 1.0 g of BPO as a thermal initiator, to obtain a polymerizable monomer composition.

(3) According to the formulation in Table 2, the modified filler, the polymerizable monomer composition, colorants and an additive were mixed to be uniform to obtain paste materials with different colors.

(4) The paste materials with different colors were respectively stirred for 20 min at a rotating speed of 80 r/min under a vacuum degree of -0.09 MPa, to obtain granular materials with an average particle size of 5 mm.

(5) The granular materials were weighted for each layer according to a color transition sequence, then introduced into a press-forming mould in sequence, and spread to be flat. A pressure of 3 MPa was applied to the mould to combine layers tight, to obtain a semi-finished product of a multilayer gradient color-resin ceramic restoration material.

(6) The semi-finished product of the multilayer gradient color-resin ceramic restoration material obtained in step (5) was pressed at 100 °C and 15 MPa for 4 h for polymerizing and curing, obtaining the multilayer gradient color-resin ceramic restoration material.

Table 2

| Number of layers | Modified glass powder | Polymerizable monomer composition | Titanium dioxide | Iron(III) oxide | Iron oxide yellow | Iron oxide black |
|---|---|---|---|---|---|---|
| 1 | 79.70147% | 19.92537% | 0.07610% | 0.18463% | 0.11217% | 0.00026% |
| 2 | 79.67859% | 19.91965% | 0.08193% | 0.19878% | 0.12077% | 0.00028% |
| 3 | 79.61006% | 19.90251% | 0.09941% | 0.24117% | 0.14652% | 0.00034% |
| 4 | 79.54164% | 19.88541% | 0.11685% | 0.28348% | 0.17223% | 0.00040% |
| 5 | 79.47333% | 19.86833% | 0.13426% | 0.32572% | 0.19789% | 0.00046% |

**Example 3**

(1) Amorphous barium boroaluminosilicate glass powder GM27884 with a D50 of 0.18 $\mu$m and fumed silica OX-50 with a D50 of 40 nm were modified by using $\gamma$-MPS, respectively, to obtain a modified glass powder and a modified fumed silica.

(2) 70 g of UDMA and 30 g of TEGDMA were added with 1.0 g of BPO as a thermal initiator, to obtain a polymerizable monomer composition.

(3) According to the formulation in Table 3, the modified filler, the polymerizable monomer composition, colorants and an additive were mixed to be uniform to obtain paste materials with different colors.

(4) The paste materials with different colors were respectively stirred for 30 min at a rotating speed of 90 r/min under a vacuum degree of -0.08 MPa, to obtain granular materials with an average particle size of 2.1 mm.

(5) The granular materials were weighted for each layer according to a color transition sequence, then introduced into a press-forming mould in sequence, and spread to be flat. A pressure of 2 MPa was applied to the mould to combine layers tight, to obtain a semi-finished product of a multilayer gradient color-resin ceramic restoration material.

(6) The semi-finished product of the multilayer gradient color-resin ceramic restoration material obtained in step (5) was pressed at 100 °C and 10 MPa for 4 h for polymerizing and curing, obtaining the multilayer gradient color-resin ceramic restoration material.

Table 3

| Number of layers | Modified glass powder | Modified fumed silica | Polymerizable monomer composition | Titanium dioxide | Iron(III) oxide | Iron oxide yellow |
|---|---|---|---|---|---|---|
| 1 | 69.82684% | 4.98763% | 24.93816% | 0.00420% | 0.14435% | 0.09882% |
| 2 | 69.75932% | 4.98281% | 24.91404% | 0.00584% | 0.20063% | 0.13735% |
| 3 | 69.69192% | 4.97799% | 24.88997% | 0.00748% | 0.25681% | 0.17581% |
| 4 | 69.62466% | 4.97319% | 24.86595% | 0.00911% | 0.31289% | 0.21420% |

**Example 4**

(1) Amorphous barium boroaluminosilicate glass powder GM27884 with a D50 of 0.18 $\mu$m was modified by using $\gamma$-MPS, to obtain a modified glass powder.

(2) 65 g of Bis-GMA and 35 g of TEGDMA were added with 1.0 g of BPO as a thermal initiator, to obtain a polymerizable monomer composition.

(3) According to the formulation in Table 4, the modified filler, the polymerizable monomer composition, colorants and an additive were mixed to be uniform to obtain paste materials with different colors.

(4) The paste materials with different colors were respectively stirred for 50 min at a rotating speed of 110 r/min under a vacuum degree of -0.08 MPa, to obtain granular materials with an average particle size of 0.1 mm.

(5) The granular materials were weighted for each layer according to a color transition sequence, then introduced into a press-forming mould in sequence, and spread to be flat. A pressure of 3 MPa was applied to the mould to combine layers tight, to obtain a semi-finished product of a multilayer gradient color-resin ceramic restoration material.

(6) The semi-finished product of the multilayer gradient color-resin ceramic restoration material obtained in step (5) was pressed at 100 °C and 15 MPa for 4 h for polymerizing and curing, obtaining the multilayer gradient color-resin ceramic restoration material.

Table 4

| Number of layers | Modified glass powder | Polymerizable monomer composition | Titanium dioxide | Iron(III) oxide | Iron oxide yellow | Iron oxide black |
|---|---|---|---|---|---|---|
| 1 | 79.70147% | 19.92537% | 0.07610% | 0.18463% | 0.11217% | 0.00026% |
| 2 | 79.67859% | 19.91965% | 0.08193% | 0.19878% | 0.12077% | 0.00028% |
| 3 | 79.61006% | 19.90251% | 0.09941% | 0.24117% | 0.14652% | 0.00034% |
| 4 | 79.54164% | 19.88541% | 0.11685% | 0.28348% | 0.17223% | 0.00040% |
| 5 | 79.47333% | 19.86833% | 0.13426% | 0.32572% | 0.19789% | 0.00046% |

**Comparative Example 1**

(1) Amorphous barium boroaluminosilicate glass powder GM27884 with a D50 of 0.18 $\mu$m and fumed silica OX-50 with a D50 of 40 nm were modified by using $\gamma$-MPS, respectively, to obtain a modified glass powder and a modified fumed silica.

(2) 70 g of UDMA and 30 g of TEGDMA were added with 1.0 g of BPO as a thermal initiator, to obtain a polymerizable monomer composition.

(3) According to the formulation in Table 3, the modified filler, the polymerizable monomer composition, colorants and an additive were mixed to be uniform to obtain paste materials with different colors.

(4) The paste materials with different colors were introduced into a press-forming mould in sequence, during which after each paste material was introduced to be a layer, a pressure of 2 MPa was applied to the mould for 1 min. The resulting semi-finished product of a multilayer gradient color-resin ceramic restoration material was subjected to polymerizing and curing at 100 °C and 10 MPa for 1 h, obtaining the multilayer gradient color-resin ceramic restoration material.

**Comparative Example 2**

(1) Amorphous barium boroaluminosilicate glass powder GM27884 with a D50 of 0.7 $\mu$m and fumed silica OX-50 with a D50 of 40 nm were modified by using $\gamma$-MPS, respectively, to obtain a modified glass powder and a modified fumed silica.

(2) 70 g of UDMA and 30 g of TEGDMA were added with 1.0 g of BPO as a thermal initiator, to obtain a polymerizable monomer composition.

(3) According to the formulation in Table 5, the modified filler, the polymerizable monomer composition, colorants and an additive were mixed to be uniform to obtain paste materials with different colors.

(4) The paste materials with different colors were simultaneously sprayed into a press-forming mould by using injectors. The resulting semi-finished product of a multilayer gradient color-resin ceramic restoration material was subjected to polymerizing and curing at 120 °C and 8 MPa for 2 h, obtaining the multilayer gradient color-resin ceramic restoration material.

Table 5

| Number of layers | Modified glass powder | Modified fumed silica | Polymeriza ble monomer composition | Titanium dioxide | Iron(III) oxide | Iron oxide yellow | Iron oxide black |
|---|---|---|---|---|---|---|---|
| 1 | 53.52895% | 10.70579% | 35.68597% | 0.00845% | 0.03672% | 0.03043% | 0.00368% |
| 2 | 53.51087% | 10.70217% | 35.67391% | 0.01204% | 0.05236% | 0.04339% | 0.00525% |
| 3 | 53.49281% | 10.69856% | 35.66188% | 0.01564% | 0.06798% | 0.05634% | 0.00681% |

Performance of dental curable compositions prepared in Examples and Comparative Examples was tested and evaluated by the following methods.

(1) Particle size test

Method: A high-definition camera was used to capture images of the prepared particles, and the images were stored in a computer. The two-dimensional images were scanned on the computer, and pixel clusters of characteristic points were measured, counted and edited to obtain particle size.

(2) Bending strength test

Method: A composite resin block was cut into test pieces with a size of 1.2*4.0*18 mm, and then surfaces of the test pieces were polished with a 2000 mesh sandpaper by wet grinding. A three-point bending test was carried out with a tensile testing machine under the conditions of a fulcrum spacing of 12 mm and a crosshead speed of 1.0 mm/min, and the average value of ten samples was evaluated.

(3) Fracture toughness

Method: A composite resin block was processed into test strips with a size of (4.0 $\pm$0.2) mm * (3.0 $\pm$ 0.2) mm * (44 $\pm$ 1) mm. A V-groove with a depth of about (1.0 $\pm$ 0.2) mm was made on a surface with a width of 3 mm. A tensile testing machine was used to test the test strips having a V-groove placed downward, under conditions of a span of 40 mm and a crosshead speed of 0.5 mm/min. Fracture load force (N) was recorded and calculated.

(4) Water absorption value and dissolution value

Method: A composite resin block was processed into samples with a diameter of $(15\pm1)$ mm and a thickness of $(1.0\pm0.2)$ mm, and the diameter and thickness of the samples were measured to calculate the sample volume V. Samples were dried at 37 °C to a constant weight, marked as $m_1$. Samples with the constant weight were soaked in pure water at 37 °C, and taken out and weighed after 7 days, and the resulting weight was marked as $m_2$. Finally, samples were put into an oven at 37 °C and dried to a constant weight, marked as $m_3$.

Water absorption value $\rho_{ws}$

$$\rho_{ws} = (m_2-m_3)/V$$

Dissolution value $\rho_{sl}$

$$\rho_{sl} = (m_1-m_3)/V$$

Results of performance test are shown in Table 6.

Table 6

| Example No. | Particle size | Bending strength | Elastic modulus | Fracture toughness | Water absorption value | Dissolution value | Color transition |
|---|---|---|---|---|---|---|---|
| | mm | MPa | GPa | MPa·m$^{1/2}$ | $\mu$g/mm$^3$ | $\mu$g/mm$^3$ | |
| Example 1 | 1.2 | 217 | 9.1 | 2.53 | 26.23 | 1.27 | Natural color transition and good gradient change effect. |
| Example 2 | 2.5 | 244 | 7.2 | 2.22 | 25.69 | 1.31 | Natural color transition and good gradient change effect. |
| Example 3 | 2.1 | 235 | 8.2 | 2.42 | 28.32 | 1.31 | Natural color transition and good gradient change effect. |
| Example 4 | 0.7 | 215 | 9.5 | 2.38 | 26.58 | 0.87 | Natural color transition and good gradient change effect. |
| Comparative example 1 | / | 190 | 8.6 | 1.95 | 26.75 | 1.73 | Obvious layering. |
| Comparative example 2 | / | 183 | 7.5 | 2.08 | 21.77 | 1.23 | No obvious layering, but poor gradient change effect. |

It can be seen from table 6 that the resin ceramic restoration material prepared according to the present invention has natural color transition without obvious layering, and good gradient change effect, and has a bending strength of 215 MPa or more, an elastic modulus of 7.2-9.5 GPa, a fracture toughness of 2.22-2.53 MPa · m$^{1/2}$, a water absorption value of 25.69-28.32 $\mu$g/mm$^3$, and a dissolution value of 0.87-1.31 $\mu$g/mm$^3$.

The applicant declares that in the present disclosure, a dental gradient color-resin ceramic restoration material and a preparation method thereof are illustrated through the above examples, but the present invention is not limited to the above examples, i.e., not meaning that the present invention is implemented only by the above examples. It should be clear to those skilled in the art that any improvement of the present disclosure, such as equivalent substitution of raw materials of the product of the present invention, addition of auxiliary ingredients, selection of specific means, should fall within the scope of protection and disclosure of the present invention.

**Claims**

1. A method for preparing a dental gradient color-resin ceramic restoration material, comprising:

   step 1, preparing at least two paste materials with different colors, and respectively dispersing the paste materials with different colors to obtain at least two granular materials with different colors;
   step 2, spreading the at least two granular materials with different colors to be flat in a mould in sequence to obtain at least two layers, and then tightly combining the at least two layers to obtain a semi-finished product of a gradient color-resin ceramic restoration material; and
   step 3, polymerizing and curing the semi-finished product obtained in the step 2 to obtain a multilayer gradient color-resin ceramic restoration material.

2. The method of claim 1, wherein in step 1, each of the paste materials is independently prepared by a process comprising:

   adding a polymerizable monomer, an initiator, an inorganic filler and a colorant into a mixing device, and stirring to be uniform to obtain the paste material; or
   adding a polymerizable monomer, an initiator, an inorganic filler and a colorant into a mixing device in batches, and stirring to be uniform to obtain the paste material; and
   preferably, the at least two granular materials with different colors obtained in step 1 each independently have a particle size of 0.1 mm to 5 mm.

3. The method of claim 1, wherein the dispersing in step 1 is conducted at a rotating speed of 20 r/min to 150 r/min, preferably 60 r/min to 120 r/min;

   preferably, the dispersing in step 1 is conducted under a vacuum degree of -0.01 MPa to -0.1MPa, preferably -0.07 MPa to -0.09 MPa;
   preferably, the tightly combining the at least two layers in step 2 is conducted at a pressure of 0.5 MPa to 5 MPa, preferably 1 MPa to 3 MPa;
   preferably, the polymerizing and curing in step 3 is conducted at a pressure greater than that of the tightly combining the at least two layers in step 2, preferably 2 MPa to 50 MPa, and further preferably 10 MPa to 30 MPa; and
   preferably, the polymerizing and curing in step 3 is conducted at a temperature of 60 °C to 200 °C, preferably 100 °C to 150 °C.

4. The method of claim 2, wherein a weight ratio of the polymerizable monomer to the inorganic filler in raw materials for preparing the at least two paste materials with different colors is in a range of 10: 90 to 90: 10, preferably 15: 85 to 40: 60;

   preferably, a weight percentage of the initiator to a total amount of the polymerizable monomer and the inorganic filler is in a range of 0.01 wt% to 10 wt%, preferably 0.1 wt% to 5 wt%; and
   preferably, a weight percentage of the colorant to a total amount of the polymerizable monomer and the inorganic filler is in a range of 0.01 wt% to 3 wt%, preferably 0.1 wt% to 1 wt%.

5. The method of any one of claims 1 to 4, wherein raw materials for preparing the at least two paste materials with different colors further comprise an additive, and the additive is preferably at least one selected from the group consisting of an accelerator, a crosslinking agent, a fluorescent agent, a polymerization inhibitor, an antibacterial agent, an ultraviolet absorber, an anti-tarnishing agent, a viscosity regulator, a wetting agent, an antioxidant, a stabilizer, and a diluent; and
   preferably, a weight percentage of the additive to a total amount of the polymerizable monomer and the inorganic filler is in a range of 0 wt% to 5 wt%, preferably 0.01 wt% to 1 wt%.

6. The method of any one of claims 1 to 3, wherein the polymerizable monomer is a free radical resin monomer, and the free radical resin monomer comprises at least one selected from the group consisting of a monofunctional group-monomer compound, a bifunctional group-monomer compound and a monomer compound with at least three functional groups, wherein

preferably, the monofunctional group-monomer compound comprises at least one selected from the group consisting of methyl methacrylate, ethyl methacrylate, isobutyl methacrylate, tert-butyl methacrylate, iso-amyl methacrylate, benzyl methacrylate, glycidyl methacrylate, dodecyl methacrylate, tetrahydrofurfuryl methacrylate, 2-(N,N-dimethylamino) ethyl methacrylate, 2,3-dibromopropyl methacrylate, 2-hydroxyethyl methacrylate, 2-hydroxypropyl methacrylate, 6-hydroxyhexyl methacrylate, 10-hydroxydecyl methacrylate, triethylene glycol mono monomethacrylate, propylene glycol monomethacrylate, ethylene glycol methacrylate, diethylene glycol methacrylate, methoxy diethylene glycol methacrylate, polyethylene glycol methacrylate, N-methylol methacrylamide, N-succinyl methyl acrylamide, and 10-methacryloyloxydecyl dihydrogen phosphate;
preferably, the bifunctional group-monomer compound comprises at least one selected from the group consisting of ethylene glycol dimethacrylate, propylene glycol dimethacrylate, neopentanediol dimethacrylate, butanediol dimethacrylate, hexanediol dimethacrylate, poly(ethylene glycol) dimethacrylate, triethylene glycol dimethacrylate, urethane dimethacrylate, bisphenol A glycerolate dimethacrylate (2,2-bis(4-(3-methacryloyloxy-2-hydroxypropoxy)phenyl)propane), 2,2-bis(4-methacryloyloxyethoxyphenyl)propane, 2,2-bis(4-methacryloyloxydiethoxyphenyl)propane, and 2,2-bis(4-methacryloyloxypolyethoxyphenyl)propane; and
preferably, the monomer compound with at least three functional groups comprises at least one selected from the group consisting of trimethylolpropane trimethacrylate, trimethylolethane trimethacrylate, trimethylolpropane trimethacrylate, tetramethylolmethane trimethacrylate, pentaerythritol tetramethylacrylate, bis-trimethylolpropane tetramethacrylate, dipentaerythritol tetramethacrylate, dipentaerythritol hexamethacrylate, and N,N'-(2,2,4-trimethylhexamethylene)bis(2-(aminocarboxyl) propane-1,3-diol) tetramethylacrylate.

7. The method of any one of claims 1 to 4, wherein the polymerizable monomer after polymerizing has a refractive index of 1.52 to 1.58, preferably 1.53 to 1.55.

8. The method of any one of claims 1 to 5, wherein the inorganic filler comprises an inorganic filler A and an inorganic filler B, wherein the inorganic filler A has an average particle size of 0.1 $\mu$m to 10 $\mu$m, and the inorganic filler B has an average particle size of 10 nm to 40 nm;

preferably, the inorganic filler A comprises at least one selected from the group consisting of silica, aluminum silicate, aluminium oxide, titanium dioxide, zirconium oxide, fluorine glass, borosilicate glass, sodium glass, barium glass, barium aluminum silicate glass, strontium or zirconium containing glass, glass ceramic, fluoroaluminosilicate glass, synthetic glass obtained by sol-gel method, fumed silica, calcium fluoride, strontium fluoride, calcium carbonate, kaolin, clay, mica, aluminum sulfate, calcium sulfate, barium sulfate, titanium oxide, calcium phosphate, hydroxyapatite, calcium hydroxide, strontium hydroxide, and zeolite;
preferably, the inorganic filler B comprises at least one selected from the group consisting of silica, zirconium oxide, titanium oxide, zirconium oxide, zinc oxide, calcium phosphate, and hydroxyapatite; and
preferably, the inorganic filler A has a refractive index of 1.52 to 1.58, and the inorganic filler B has a refractive index of 1.43 to 1.50.

9. The method of any one of claims 1 to 8, wherein the inorganic filler is a surface treated inorganic filler using a surface treatment agent;

preferably, the surface treatment agent comprises at least one selected from the group consisting of $\gamma$-methacryloxy propyl trimethoxyl silane, $\gamma$-methacryloxypropyltriethoxysilane, and $\gamma$-aminopropyltrimethoxysilane, and preferably $\gamma$-methacryloxy propyl trimethoxyl silane;
preferably, the surface treatment agent is used in an amount of 0.1-50 parts by weight, preferably 1-30 parts by weight, based on 100 parts by weight of the inorganic filler;
preferably, the initiator is at least one selected from the group consisting of dicumyl peroxide, tert-butyl peroxide, benzoyl peroxide, tert-butyl peroxyacetate, and tert-butyl peroxybenzoate; and
preferably, the colorant is at least one selected from the group consisting of zinc white, titanium dioxide, antimony oxide, vermilion, cadmium red, iron(III) oxide, chromium oxide, cerium praseodymium yellow, lemon yellow, lead sulfochromate yellow, zinc chrome yellow, iron oxide yellow, bismuth yellow, barium chromate, zirconium vanadium yellow, iron oxide black, carbon black, and graphite.

10. A dental gradient color-resin ceramic restoration material prepared by the method of any one of claims 1 to 9.

Europäisches
Patentamt

European
Patent Office

Office européen
des brevets

# EUROPEAN SEARCH REPORT

Application Number

**EP 22 19 2422**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 689 322 A1 (LIAONING UPCERA CO LTD [CN]) 5 August 2020 (2020-08-05) * paragraphs [0077], [0084] – [0111]; examples 1-6; table 1 * | 1-10 | INV. A61K6/15 A61K6/78 A61K6/836 A61K6/887 |
| X,D | EP 3 287 118 A1 (SHENZHEN UPCERA DENTAL TECH CO LTD [CN]) 28 February 2018 (2018-02-28) * paragraphs [0018] – [0025]; examples 1-9; tables 1,2 * | 1-10 | A61K6/64 A61K6/76 A61K6/77 A61K6/17 |
| X | WO 2021/132861 A1 (VERICOM CO LTD [KR]) 1 July 2021 (2021-07-01) * paragraphs [0091] – [0120]; examples 1-3; table 1 * | 1-10 | |
| X,D | WO 2009/154301 A1 (ADVANCE KK [JP]; KONTANI KAZUO [JP]; EGUCHI MIKI [JP]) 23 December 2009 (2009-12-23) * page 51, line 25 – page 58, line 10; examples 2,4; tables 1-4 * | 1-10 | TECHNICAL FIELDS SEARCHED (IPC) A61K |

The present search report has been drawn up for all claims

| Place of search | Date of completion of the search | Examiner |
|---|---|---|
| The Hague | 12 January 2023 | Barenbrug-van Druten |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
............................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 22 19 2422

This annex lists the patent family members relating to the patent documents cited in the above-mentioned European search report.
The members are as contained in the European Patent Office EDP file on
The European Patent Office is in no way liable for these particulars which are merely given for the purpose of information.

12-01-2023

| Patent document cited in search report | | Publication date | Patent family member(s) | | Publication date |
|---|---|---|---|---|---|
| EP 3689322 | A1 | 05-08-2020 | AU | 2018340731 A1 | 07-05-2020 |
| | | | CN | 109589270 A | 09-04-2019 |
| | | | EP | 3689322 A1 | 05-08-2020 |
| | | | JP | 6953626 B2 | 27-10-2021 |
| | | | JP | 2020536061 A | 10-12-2020 |
| | | | KR | 20200060756 A | 01-06-2020 |
| | | | US | 2020268616 A1 | 27-08-2020 |
| | | | WO | 2019062144 A1 | 04-04-2019 |
| EP 3287118 | A1 | 28-02-2018 | CN | 105362084 A | 02-03-2016 |
| | | | EP | 3287118 A1 | 28-02-2018 |
| | | | ES | 2903229 T3 | 31-03-2022 |
| | | | JP | 6633752 B2 | 22-01-2020 |
| | | | JP | 2018522066 A | 09-08-2018 |
| | | | KR | 20180014430 A | 08-02-2018 |
| | | | US | 2018161251 A1 | 14-06-2018 |
| | | | WO | 2017080092 A1 | 18-05-2017 |
| WO 2021132861 | A1 | 01-07-2021 | KR | 20210083623 A | 07-07-2021 |
| | | | WO | 2021132861 A1 | 01-07-2021 |
| WO 2009154301 | A1 | 23-12-2009 | JP | WO2009154301 A1 | 01-12-2011 |
| | | | WO | 2009154301 A1 | 23-12-2009 |

EPO FORM P0459

For more details about this annex : see Official Journal of the European Patent Office, No. 12/82

**EP 4 144 334 A1**

**REFERENCES CITED IN THE DESCRIPTION**

**Patent documents cited in the description**

- WO 2009154301 A1
- WO 2018074583 A1
- CN 105362084 A